# EUROPÄISCHE PATENTANMELDUNG

(11) **EP 1 413 566 A1**
(43) Veröffentlichungstag der Anmeldung: **28.04.2004**
(21) Anmeldenummer: 03023176.5
(22) Anmeldetag: 13.10.2003
(51) Int. Cl.: C07C 17/363, C07C 25/13, C07C 25/02

(54) **Verfahren zur Herstellung 1,3-Dihalogen-substituierten Benzolderivaten**

(30) Priorität: 25.10.2002 DE 10249748
(71) Anmelder: Clariant GmbH, 65929 Frankfurt am Main (DE)
(72) Erfinder: Brand, Stefan, Dr., 69198 Schriesheim (DE); Decker, Daniel, Dr., 65835 Liederach a. Ts. (DE); Wessel, Thomas, Dr., 61138 Niederdorfelden (DE)
(74) Vertreter: Clariant Service GmbH

(57) **Zusammenfassung**

Verfahren zur Herstellung von 1,3-Dihalogen-substituierten Benzolderivaten (II) aus 2,6-Dihalogen-substituierten Benzaldehyden (I) (mit X₁, X₂ = unabhängig voneinander F, Cl, Br und R₁, R₂, R₃ = unabhängig voneinander H, Halogen, OH, C₁-C₁₂-Alkyl, CF₃, CHO, C₆-C₁₄-Aryl, OAlkyl, OAryl, NO₂) durch Umsetzung mit einem alkalisch wirkenden Medium, dadurch gekennzeichnet, dass das alkalisch wirkende Medium vorgelegt wird und der 2,6-Dihalogen-substituierte Benzaldehyd (I) zudosiert wird oder der 2,6-Dihalogen-substituierte Benzaldehyd (I) vorgelegt wird und das alkalisch wirkende Medium zudosiert wird.

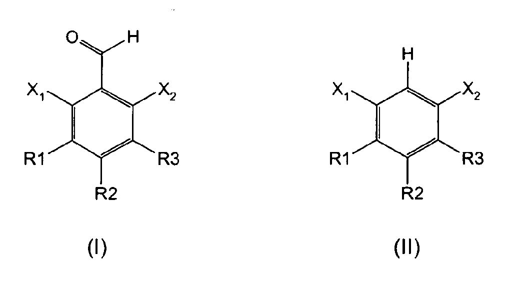

## Beschreibung

Die vorliegende Erfindung betrifft ein verbessertes Verfahren zur Herstellung von 1,3-Dihalogen-substituierten Benzolderivaten (II) aus 2,6-Dihalogen-substituierten Benzaldehyden (I) durch Umsetzung mit einem alkalisch wirkenden Medium, wobei das alkalisch wirkende Medium vorgelegt wird und der 2,6-Dihalogen-substituierte Benzaldehyd (I) zudosiert wird oder der 2,6-Dihalogen-substituierte Benzaldehyd (I) vorgelegt wird und das alkalisch wirkende Medium zudosiert wird.

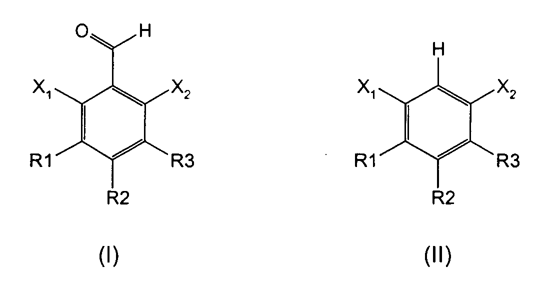

Insbesondere betrifft diese Erfindung ein gegenüber dem Stand der Technik verbessertes Verfahren, bei dem 1,3-Dihalogen-substituierte Benzolderivate (II) in hoher Selektivität und Ausbeute erhalten werden und die Übertragung in den Produktionsmaßstab sicherheitstechnische Anforderungen erfüllt.

1,3-Dihalogen-substituierte Benzolderivate (z.B. 1,3-Difluorbenzol) sind wichtige Zwischenprodukte zur Herstellung pharmazeutischer und agrochemischer Produkte.

Verfahren zur Herstellung von 1,3-Dihalogen-substituierten Benzolderivaten (II) sind an sich bekannt. So kann z.B. 1,3-Difluorbenzol durch katalytische Abspaltung von Chlor ausgehend von 2,4-Difluorchlorbenzol synthetisiert werden (EP-A-598 338). Nachteilig bei dieser Verfahrensweise ist, dass 2,4-Difluorchlorbenzol nur durch in technischer Hinsicht schwierige und aufwendige Verfahrensschritte zugänglich ist, z.B. durch Chlor/Fluor-Austausch ausgehend von 2,4-Dichlornitrobenzol, gefolgt von denitrierender Chlorierung des resultierenden 2,4-Difluornitrobenzols (EP-A-598 338).

Als Alternative zur Herstellung von 1,3-Dihalogen-substituierten Benzolderivaten (II) ist es vorteilhaft, 2,6-dihalogen-substituierte Benzaldehyde (I) als Ausgangsmaterialien einzusetzen. So ist z.B. 2,6-Difluorbenzaldehyd im Falle von 1,3-Difluorbenzol in hoher Ausbeute ausgehend von 2,6-Dichlorbenzaldehyd durch Chlor/Fluoraustausch zugänglich (EP-A-1 070 724).

Es ist bereits bekannt, dass 1,3-Dihalogen-substituierte Benzolderivate (II) durch Umsetzung von 2,6-Dihalogen-substituierten Benzaldehyden (I) mit wässriger Alkalilauge hergestellt werden können (Lock, Chem. Ber. 1936, 69, 2253). Die beschriebene Vorgehensweise hat jedoch den Nachteil, dass alle Einsatzstoffe auf einmal vorgelegt werden und anschließend das Reaktionsgemisch bis zur erforderlichen Reaktionstemperatur erwärmt wird. Dabei wird die Entstehung eines Feststoffs beschrieben und auch selbst beobachtet (s. Vergleichsbeispiele), was negative Auswirkungen auf die Selektivität und die Ausbeute der Umsetzung hat. So wird im Falle der Synthese von 1,3-Difluorbenzol aus 2,6-Difluorbenzaldehyd nur eine mäßige Ausbeute von 61 % beschrieben (Lock, Chem. Ber. 1936, 69, 2253). Die mäßige Ausbeute kann auch durch Vergleichsversuche bestätigt werden (s. Vergleichsbeispiele). Ein weiterer Nachteil besteht darin, dass das gleichzeitige Vorlegen aller Einsatzstoffe vor Beginn der Reaktion bei der Übertragung in den Produktionsmaßstab aus sicherheitstechnischen Gründen problematisch und somit nicht realisierbar ist.

Im Hinblick auf die vorstehend erwähnten Nachteile der bekannten Verfahren, besteht der Bedarf, ein verbessertes Verfahren bereitzustellen, das alle diese Nachteile nicht aufweist.

Diese Aufgabe wird gelöst durch ein Verfahren zur Herstellung von 1,3-Dihalogen-substituierten Benzolderivaten (II) aus 2,6-Dihalogen-substituierten Benzaldehyden (I) (mit X₁, X₂ = unabhängig voneinander F, Cl, Br und R₁, R₂, R₃ = unabhängig voneinander H, Halogen, insbesondere F, Cl, Br, OH, C₁-C₁₂-Alkyl, insbesondere C₁-C₈-Alkyl-, CF₃, CHO, C₆-C₁₄-Aryl, OAlkyl, OAryl, NO₂) durch Umsetzung mit einem alkalisch wirkenden Medium, dadurch gekennzeichnet, dass das alkalisch wirkende Medium vorgelegt wird und der 2,6-Dihalogen-substituierte Benzaldehyd (I) zudosiert wird oder der 2,6-Dihalogen-substituierte Benzaldehyd (I) vorgelegt wird und das alkalisch wirkende Medium zudosiert wird.

Als erfindungsgemäß einsetzbare 2,6-Dihalogen-substituierte Benzaldehyde (I) seien beispielsweise genannt: 2,6-Difluorbenzaldehyd, 2-Chlor-6-fluorbenzaldehyd, Tetrafluorterephthalaldehyd, 2,4,6-Trifluorbenzaldehyd, 4-Chlor-2,6-difluorbenzaldehyd, 2,4-Dichlor-6-fluorbenzaldehyd, Pentafluorbenzaldehyd, 3,5-Dichlor-2,4,6-trifluorbenzaldehyd, 2,4,5,6-Tetrafluorbenzol-1,3-dicarbaldehyd oder 5-Chlor-2,4,6-trifluorbenzol-1,3-dicarbaldehyd, um nur einige zu nennen. Besonders bevorzugt werden 2,6-Difluorbenzaldehyd, 2-Chlor-6-fluorbenzaldehyd und Tetrafluorterephthalaldehyd.

Es ist zweckmäßig, die Umsetzung, wegen der Empfindlichkeit von Benzaldehyden gegenüber Luftsauerstoff, unter Schutzgas (z.B. Argon oder Stickstoff) durchzuführen.

Als alkalisch wirkendes Medium können wässrige Lösungen von Basen der Alkali- oder Erdalkalimetalle, insbesondere Alkali- oder Erdalkalihydroxyd- oder -carbonat-Lösungen, beispielsweise Natriumhydroxid, Kaliumhydroxid, Calciumhydroxid, Natriumcarbonat, Kaliumcarbonat oder auch Ammoniak verwendet werden, besonders bevorzugt werden wässrige Lösungen von Natriumhydroxid, Kaliumhydroxid und Calciumhydroxid.

Die Abspaltung der Carbonylgruppe erfolgt derart, dass unter Einwirkung des alkalisch wirkenden Mediums pro Mol an Carbonylgruppe ein Mol Formiat entsteht. Das alkalisch wirkende Medium wird daher in einer Menge eingesetzt, dass für jedes Mol an abzuspaltender Carbonylgruppe der 2,6-Dihalogen-substituierten Benzaldehyde (I) mindestens ein Mol der oben genannten Basen eingesetzt wird.

Die Konzentration des eingesetzten alkalisch wirkenden Mediums kann frei gewählt werden, sollte aber nicht zu klein sein. Im Falle der besonders bevorzugten Basen Natriumhydroxid und Kaliumhydroxid ist es zweckmäßig, eine Basenkonzentration im Bereich von 40 bis 50 Gew.-% zu wählen.

Das Verfahren kann im Temperaturbereich von 50 bis 215°C durchgeführt werden, bevorzugt wird der Temperaturbereich von 70 bis 160°C, besonders bevorzugt wird der Temperaturbereich von 70 bis 110°C.

Die Isolierung der 1,3-Dihalogen-substituierten Benzolderivate (II) kann beispielsweise durch Wasserdampfdestillation, durch Extraktion mit einem mit Wasser nicht mischbaren Lösungsmittel (z.B. Diethylether, Methyl-tert.-butylether, Toluol, Xylol), durch Kristallisation oder durch chromatographische Methoden erfolgen. Bei Reaktionsprodukten mit einem Siedepunkt kleiner als jenem von Wasser ist das gleichzeitige Abdestillieren der Reaktionsprodukte während der dosier-kontrollierten Umsetzung möglich. Zur Erreichung sehr reiner 1,3-Dihalogensubstituierter Benzolderivate (II) ist die anschließende Durchführung einer fraktionierten Destillation zweckmäßig.

Es ist als sehr überraschend anzusehen, dass bei der Durchführung des erfindungsgemäßen Verfahrens die 1,3-Dihalogen-substituierten Benzolderivate (II) selektiver und in sehr viel höherer Ausbeute erhalten werden als bei der dem Stand der Technik entsprechenden Verfahrensweise. So sind mit dem erfindungsgemäßen Verfahren Ausbeuten > 80 %, insbesondere Ausbeuten > 90 % erreichbar. Das erfindungsgemäße Verfahren hat insbesondere und unter anderem den Vorteil, dass die Umsetzung von 2,6-Dihalogen-substituierten Benzaldehyden (I) zu 1,3-Dihalogen-substituierten Benzolderivaten (II) über die Dosiergeschwindigkeit kontrolliert werden und die Reaktion somit jederzeit unterbrochen werden kann. Ferner werden bei der Übertragung des Verfahrens in den Produktionsmaßstab die sicherheitstechnischen Anforderungen erfüllt.

Weiterhin ist es sehr überraschend, dass diese Vorgehensweise sogar auf komplizierte Substitutionsmuster, d.h. Dicarbonylverbindungen, übertragbar ist, wie z.B. Tetrafluorterephthalaldehyd, die mit den im Stand der Technik bekannten Verfahren nur mit schlechten Ausbeuten erhalten werden können. Bei einer Umsetzung gemäß des erfindungsgemäßen Verfahrens werden jedoch sehr gute Ausbeuten (siehe Beispiel 4) erreicht, wobei ferner die Rohprodukte ebenfalls in hoher Reinheit anfallen.

Die nachfolgenden Beispiele und Vergleichsbeispiele dienen zur Veranschaulichung des Gegenstands der Erfindung, ohne dass die Erfindung auf diese Beispiele beschränkt sein soll.

### Beispiele

### Beispiel 1: Synthese von 1,3-Difluorbenzol aus 2,6-Difluorbenzaldehyd

In einen Glaskolben mit Rührer, Thermometer, Tropftrichter und Destillationsbrücke werden bei Raumtemperatur und unter Argon 245 g Wasser vorgelegt und unter Rühren 245 g (3,80 mol) KOH-Prills (87 %ig) zugegeben. Nachdem sich die KOH-Prills vollständig gelöst haben, wird auf eine Innentemperatur von 95°C erwärmt. Innerhalb von 3 bis 4 Stunden werden 500 g (3,52 mol) 2,6-Difluorbenzaldehyd zugetropft. Dabei destilliert 1,3-Difluorbenzol zusammen mit einer kleinen Menge an Wasser ab und wird in einer mit Eis gekühlten Vorlage aufgefangen. Anschließend wird noch 15 min auf 120°C Innentemperatur erwärmt, um letzte Reste an 1,3-Difluorbenzol abzudestillieren. Das Destillat wird einer Phasentrennung unterzogen, wobei 380 g (3,33 mol) 1,3-Difluorbenzol erhalten werden (Ausbeute 94,6 %). Der Gehalt an 1,3-Difluorbenzol wird gaschromatographisch ermittelt und ist größer als 99,0 %.

Beispiel 2, Vergleichsbeispiele (vgl. Lock, Chem. Ber. 1936, 69, 2253). Alle Einsatzstoffe werden auf einmal vorgelegt.

### a) Mengenverhältnisse wie Lock, Chem. Ber. 1936, 69, 2253.

In einen Glaskolben mit Rührer, Thermometer, Tropftrichter und Destillationsbrücke werden unter Eiskühlung und unter Argon 50 g (0,352 mol) 2,6-Difluorbenzaldehyd sowie 345 g (3,07 mol) wässrige KOH-Lösung (50 %ig) vorgelegt, wobei die Bildung eines gelben Feststoffs beobachtet wird. Anschließend wird die Reaktionsmischung auf 95 bis100°C erwärmt, wobei 1,3-Difluorbenzol abdestilliert und in einer gekühlten Vorlage aufgefangen wird. Das Destillat wird einer Phasentrennung unterzogen, wobei 21,0 g (0,184 mol) 1,3-Difluorbenzol erhalten werden (Ausbeute 52,3 %). Im verbleibenden Reaktionsgemisch werden vergleichsweise große Mengen eines gelben Feststoffs beobachtet, der nicht weiter charakterisiert werden konnte.

### b) Mengenverhältnisse wie Beispiel 1

In einen Glaskolben mit Rührer, Thermometer, Tropftrichter und Destillationsbrücke werden bei Raumtemperatur und unter Argon 49 g (0,380 mol) wässrige KOH-Lösung (43,5 %ig) sowie 50 g (0,352 mol) 2,6-Difluorbenzaldehyd vorgelegt, wobei ein gelber Feststoff entsteht. Anschließend wird die Reaktionsmischung auf 95°C erwärmt, wobei 1,3-Difluorbenzol abdestilliert und in einer gekühlten Vorlage aufgefangen wird. Anschließend wird noch 15 min auf 120°C Innentemperatur erwärmt, um letzte Reste an 1,3-Difluorbenzol abzudestillieren. Das Destillat wird einer Phasentrennung unterzogen, wobei 24,1 g (0,211 mol) 1,3-Difluorbenzol erhalten werden (Ausbeute 59,9 %). Im verbleibenden Reaktionsgemisch werden vergleichsweise große Mengen eines gelben Feststoffs beobachtet, der nicht weiter charakterisiert werden konnte.

### Beispiel 3: Synthese von 1-Chlor-3-fluorbenzol aus 2-Chlor-6-fluorbenzaldehyd

In einem Glaskolben mit Rückflusskühler, Rührer und Thermometer werden 36 g (0,289 mol) wässrige KOH-Lösung (45 %ig) unter Argon auf 95°C erwärmt. Unter Rühren werden 41 g (0,259 mol) 2-Chlor-6-fluorbenzaldehyd innerhalb von 2 bis 3 Stunden in kleinen Portionen (jeweils ca. 2 g) zugegeben. Nach Beendigung der Zugabe wird 15 min bei 95°C nachgerührt und auf Raumtemperatur abgekühlt. Gaschromatographisch wird nach Extraktion einer Probe des Reaktionsgemischs mit Methyl-tert.-butylether vollständiger Umsatz an 2-Chlor-6-fluorbenzaldehyd beobachtet. Nach Extraktion des Reaktionsgemischs mit Methyl-tert.-butylether, Trocknung der organischen Phase über Na₂SO₄ und fraktionierte Destillation werden 31 g (0,238 mol) 1-Chlor-3-fluorbenzol (Ausbeute 91,9 %) mit einem Gehalt (GC) > 99,0 % erhalten.

### Beispiel 4: Synthese von 1,2,4,5-Tetrafluorbenzol aus Tetrafluorterephthalaldehyd

In einem Glaskolben mit Rückflusskühler, Rührer und Thermometer werden 76 g (0,610 mol) wässrige KOH-Lösung (45 %ig) unter Argon auf 90 bis 95°C erwärmt. Unter Rühren werden 42 g (0,204 mol) Tetrafluorterephthalaldehyd innerhalb von 3 Stunden in kleinen Portionen (jeweils ca. 2 g) zugegeben. Nach Beendigung der Zugabe wird 15 min bei 95°C nachgerührt und auf Raumtemperatur abgekühlt. Gaschromatographisch wird nach Extraktion einer Probe des Reaktionsgemischs mit Diethylether vollständiger Umsatz an Tetrafluorterephthalaldehyd detektiert. Nach Extraktion mit Diethylether, Trocknung der organischen Phase über Na₂SO₄ und fraktionierter Destillation werden 25,1 g (0,167 mol) 1,2,4,5-Tetrafluorbenzol (Ausbeute 81,9 %) erhalten.

## Patentansprüche

1. Verfahren zur Herstellung von 1,3-dihalogen-substituierten Benzolderivaten (II) aus 2,6-dihalogen-substituierten Benzaldehyden (I) (mit X₁, X₂ = unabhängig voneinander F, Cl, Br und R₁, R₂, R₃ = unabhängig voneinander H, Halogen, OH, C₁-C₁₂-Alkyl, CF₃, CHO, C₆-C₁₄-Aryl, OAlkyl, OAryl, NO₂) durch Umsetzung mit einem alkalisch wirkenden Medium, **dadurch gekennzeichnet, dass** das alkalisch wirkende Medium vorgelegt wird und der 2,6-Dihalogen-substituierte Benzaldehyd (I) zudosiert wird oder der 2,6-Dihalogen-substituierte Benzaldehyd (I) vorgelegt wird und das alkalisch wirkende Medium zudosiert wird.

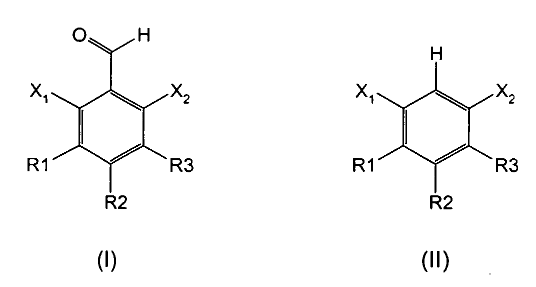

2. Verfahren nach Anspruch 1, **dadurch gekennzeichnet, dass** als 2,6-Dihalogen-substituierte Benzaldehyde (I) 2,6-Difluorbenzaldehyd, 2-Chlor-6-fluorbenzaldehyd, Tetrafluorterephthalaldehyd, 2,4,6-Trifluorbenzaldehyd, 4-Chlor-2,6-difluorbenzaldehyd, 2,4-Dichlor-6-fluorbenzaldehyd, Pentafluorbenzaldehyd, 3,5-Dichlor-2,4,6-trifluorbenzaldehyd, 2,4,5,6-Tetrafluorbenzol-1,3-dicarbaldehyd oder 5-Chlor-2,4,6-trifluorbenzol-1,3-dicarbaldehyd eingesetzt werden.

3. Verfahren nach Anspruch 2, **dadurch gekennzeichnet, dass** als 2,6-Dihalogen-substituierte Benzaldehyde (I) 2,6-Difluorbenzaldehyd, 2-Chlor-6-fluorbenzaldehyd oder Tetrafluorterephthalaldehyd eingesetzt werden.

4. Verfahren nach mindestens einem der Ansprüche 1 bis 3, **dadurch gekennzeichnet, dass** als alkalisch wirkendes Medium eine wässrige Alkali- oder Erdalkalihydroxyd oder -carbonat-Lösung verwendet wird.

5. Verfahren nach mindestens einem der Ansprüche 1 bis 4, **dadurch gekennzeichnet, dass** die Umsetzung in einem Temperaturbereich von 50 - 215°C durchgeführt wird.

6. Verfahren nach mindestens einem der Ansprüche 1 bis 5, **dadurch gekennzeichnet, dass** die Umsetzung in einem Temperaturbereich von 70 - 160°C durchgeführt wird.

7. Verfahren nach mindestens einem der Ansprüche 1 bis 6, **dadurch gekennzeichnet, dass** die Umsetzung unter Schutzgas durchgeführt wird.

8. Verfahren nach mindestens einem der Ansprüche 1 bis 7, **dadurch gekennzeichnet, dass** die Konzentration des alkalisch wirkenden Mediums im Bereich von 40 bis 50 Gew.-% liegt.

9. Verfahren nach mindestens einem der Ansprüche 1 bis 8, **dadurch gekennzeichnet, dass** die Ausbeuten an den Derivaten der Formel (II) > 80 % sind.
